Europäisches Patentamt

European Patent Office

Office européen des brevets

(19)

(11) Numéro de publication : **0 112 240**
**B1**

(12) **FASCICULE DE BREVET EUROPÉEN**

(45) Date de publication du fascicule du brevet :
26.03.86

(51) Int. Cl.⁴ : **B 01 J 23/86**, B 01 J 23/10,
C 07 C   5/32, C 07 C 15/46

(21) Numéro de dépôt : **83402347.5**

(22) Date de dépôt : **06.12.83**

(54) **Procédé de préparation d'un catalyseur contenant des oxydes de fer, de chrome, de potassium et d'au moins un métal de terre rare, utilisable dans des réactions de déshydrogénation.**

(30) Priorité : 14.12.82 FR 8221085
14.12.82 FR 8221086

(43) Date de publication de la demande :
27.06.84 Bulletin 84/26

(45) Mention de la délivrance du brevet :
26.03.86 Bulletin 86/13

(84) Etats contractants désignés :
DE IT NL

(56) Documents cités :
FR-A- 2 387 200
US-A- 3 595 809
US-A- 4 134 858

(73) Titulaire : **INSTITUT FRANCAIS DU PETROLE**
**4, Avenue de Bois-Préau**
**F-92502 Rueil-Malmaison (FR)**

(72) Inventeur : **Courty, Philippe**
**91, rue Condorcet**
**F-78800 Houilles (FR)**
Inventeur : **Roussel, Michel**
**45, avenue Guillebaud**
**F-92160 Antony (FR)**
Inventeur : **Leporq, Serge**
**6, rue Villandry**
**F-78200 Mantes la Ville (FR)**
Inventeur : **Le Page, Jean-François**
**13, rue des Primevères**
**F-92500 Rueil Malmaison (FR)**
Inventeur : **Varin, Philippe**
**6, rue de la Saussaye**
**F-91300 Massy (FR)**

## Description

La présente invention concerne un procédé de préparation de catalyseurs utilisables dans des réactions de déshydrogénation et en particulier dans la déshydrogénation des hydrocarbures aliphatiques saturés ou monoéthyléniques à faible poids moléculaire (contenant par exemple de 2 à 8 atomes de carbone), ainsi que dans la déshydrogénation des hydrocarbures alkylaromatiques (tels que l'éthylbenzène ou les diéthylbenzènes), en hydrocarbures aromatiques vinyliques (tels que le styrène ou le divinylbenzène). Elle concerne également les catalyseurs obtenus par ce procédé et leur utilisation dans les réactions de déshydrogénation.

Il est connu que, dans la déshydrogénation des hydrocarbures précités, on fait passer l'hydrocarbure, de préférence additionné d'une forte proportion de vapeur d'eau (1 à 30 moles $H_2O$/mole hydrocarbure), sur un catalyseur à une vitesse volumétrique horaire, rapportée à l'hydrocarbure liquide, de 0,05 à 5, de préférence de 0,1 à 1 volume (TPN), par volume de catalyseur et par heure et à une température d'environ 450 à 750 °C.

On a déjà décrit dans l'art antérieur des catalyseurs pour la déshydrogénation d'hydrocarbures oléfiniques, tels que les butènes (en butadiène) ou d'hydrocarbures alkylaromatiques, tels que l'éthyl benzène (en styrène), ces catalyseurs renfermant une proportion pondérale majeure d'oxyde de fer, un composé (oxyde ou carbonate) de potassium, de l'oxyde de vanadium, éventuellement de l'oxyde de chrome, ainsi qu'une faible proportion (de 0,01 à 10 % en poids) d'au moins un oxyde additionnel d'un métal tel que l'aluminium, le cadmium, le cuivre, le magnésium, le manganèse, le nickel, les métaux des terres rares, l'uranium et le zinc. De tels catalyseurs sont décrits notamment dans le brevet français 2 387 200 qui correspond en substance aux brevets des Etats-Unis 4 143 083 et 4 152 300. Dans ces brevets, les métaux des terres rares sont définis comme étant les métaux de numéros atomiques de 58 à 71 inclus, c'est-à-dire, du cérium au lutétium. Dans les exemples, les métaux des terres rares utilisés sont le cérium, le praséodyme et le néodyme.

D'une manière générale, ces catalyseurs sont préparés par mélange de proportions appropriées de composés des métaux entrant dans leur composition. Cette opération consiste plus particulièrement en un malaxage en présence d'eau, permettant de former une pâte qui est ensuite éventuellement mise en forme (par exemple par extrusion), séchée et activée thermiquement.

Il a également été décrit, dans le brevet français 2 270 003, auquel correspond en substance le brevet des Etats-Unis 4 134 858, la mise en jeu d'un matériau argileux dans la fabrication d'un catalyseur de déshydrogénation principalement à base d'oxydes de fer, de chrome et de potassium. Lors de l'activation thermique, à une température convenable, généralement comprise entre 850 et 1 100 °C, le matériau argileux se combine à l'oxyde de potassium pour former un silicate double d'aluminium et de potassium (plus particulièrement de la kaliophyllite). Cette technique permet notamment de diminuer la densité de remplissage du catalyseur.

On a maintenant découvert qu'il était possible d'améliorer les propriétés catalytiques des catalyseurs renfermant, sous forme d'oxydes, du fer, du chrome et du potassium, ainsi qu'un métal de terre rare, par un procédé en deux phases tel qu'il sera décrit plus loin.

L'invention propose donc un procédé de préparation de catalyseurs qui renferment, sous forme d'oxydes, du fer, du chrome et du potassium, en particulier dans les rapports pondéraux suivants :

$$\frac{Fe_2O_3}{K_2O} \text{ de 1/1 à 10/1, de préférence de 2/1 à 7/1 ;}$$

$$\frac{CrO_3}{K_2O} \text{ de 0,05/1 à 0,4/1, de préférence de 0,1/1 à 0,3/1 ; et}$$

$$\frac{Fe_2O_3}{CrO_3} \text{ de 15/1 à 40/1, de préférence de 25/1 à 35/1 ;}$$

éventuellement un silicate double d'aluminium et de potassium, dont la composition est plus particulièrement $Al_2O_3$, 2 $SiO_2$, $K_2O$ (kaliophyllite), en une proportion de 5 à 40 % en poids ; et un métal de terre rare en une proportion, comptée en oxyde, de 1 à 15 % en poids, de préférence de 3 à 10 % en poids.

Lorsque le catalyseur renferme un silicate double d'aluminium et de potassium, il contient une quantité d'oxyde de potassium en excès par rapport à la quantité combinée sous forme de silicate double.

Dans le cas, l'excès de potassium, compté en oxyde, est avantageusement avec l'oxyde de fer et l'oxyde de chrome dans des rapports pondéraux suivants :

$$\frac{Fe_2O_3}{\text{excès de } K_2O} \text{ : de 3/1 à 10/1 ; et}$$

$$\frac{CrO_3}{\text{excès de } K_2O} \text{ : de 0,1/1 à 0,4/1.}$$

Par terre rare, on entend dans l'invention les éléments du groupe III B̂ de la classification périodique ; et plus particulièrement, le scandium, l'yttrium et les lanthanides, dont le numéro atomique va de 57 à 71 inclus.

D'une manière générale, le procédé de préparation de catalyseurs selon l'invention comprend :

— une première phase, au cours de laquelle on prépare un produit par réaction d'au moins un composé de métal de terre rare (TR) avec au moins un composé d'au moins un métal (M) choisi parmi le fer, le chrome, le cobalt, l'aluminium et le vanadium, cette réaction étant suivie d'un chauffage à température élevée ; et

— une seconde phase, au cours de laquelle on constitue un mélange comprenant de l'eau, au moins un composé de fer, au moins un composé du chrome, au moins un composé de potassium et le produit préparé à l'issue de la première phase, les divers composés de métaux étant mis en jeu dans des proportions correspondant aux proportions pondérales d'oxydes indiquées, on malaxe ledit mélange pour former une pâte qui est ensuite séchée et on active thermiquement le catalyseur par chauffage à température élevée.

Dans la première phase du procédé de l'invention, les composés métalliques peuvent être mis en jeu en des proportions correspondant à des rapports atomiques du métal de terre rare (TR) au métal M associé qui peuvent aller par exemple de 0,5/1 à 2/1. Ce rapport est le plus souvent voisin de 1/1. A l'issue de la première phase, le produit obtenu est au moins en partie, sous la forme d'un oxyde mixte de type perovskite. US-A-3595809 décrit un catalyseur à base d'une ferrite de formule $La_xCr_yFe_zO_3$, ayant une structure de type perovskite mais à proportions métalliques différentes de celles qui sont décrites dans la présente invention.

Cette première phase peut être effectuée par réaction en solution, essentiellement dans le cas où l'on associe au moins un métal de terre rare au fer, au chrome, au cobalt et/ou à l'aluminium. On peut alors utiliser tous les composés de ces éléments qui sont solubles dans l'eau, en particulier les sels d'acides minéraux ou organiques de ces éléments. Le précipité que l'on obtient dans les conditions (notamment de PH) convenables est d'abord lavé ; puis on peut le laisser mûrir quelques heures, par exemple à une température d'environ 60 à 90 °C ; on le filtre et on le sèche à des températures pouvant s'élever graduellement, par exemple de 40 à 150 °C, puis il est chauffé à une température plus élevée, allant par exemple de 400 à 950 °C. C'est pendant ce chauffage que l'on forme au moins en partie un oxyde mixte de type perovskite.

On peut encore opérer par réaction entre oxydes ou entre espèces thermiquement décomposables en oxydes, d'au moins un métal de terre rare d'une part et d'au moins un des métaux M d'autre part. Dans le cas du vanadium, mis en jeu sous la forme d'un composé de vanadium 5+, on opère sous atmosphère réductrice (hydrogène) à une température élevée, par exemple de 600 à 900 °C. Dans tous les cas, on procède ensuite à une calcination à une température de 900 à 1 250 °C.

Dans la seconde phase du procédé de l'invention, le produit issu de la première phase est mélangé aux autres ingrédients et à l'eau. Les proportions des composés du fer, du chrome et du potassium, du métal de terre rare, comptés en oxydes, correspondent plus particulièrement aux rapports pondéraux suivants :

$$\frac{Fe_2O_3}{K_2O} \quad \text{de 1/1 à 10/1, de préférence de 2/1 à 7/1,}$$

$$\frac{CrO_3}{K_2O} \quad \text{de 0,05/1 à 0,4/1, de préférence de 0,1/1 à 0,3/1, et}$$

$$\frac{Fe_2O_3}{CrO_3} \quad \text{de 15/1 à 40/1, de préférence de 25/1 à 35/1,}$$

le composé de métal de terre rare constituant, compté en oxyde, de 1 à 15 %, de préférence de 3 à 10 % en poids par rapport à l'ensemble des réactifs comptés en oxydes.

Lorsque l'on met en jeu un matériau argileux, celui-ci représente en poids une proportion d'environ 3,5 à 30 % par rapport à l'ensemble des ingrédients mis en jeu, comptés en oxydes ; le composé du potassium étant mis en jeu en excès par rapport à la quantité susceptible de se combiner audit matériau argileux, l'excès de potassium étant avec le fer et le chrome, comptés en oxydes, dans les rapports pondéraux suivants :

$$\frac{Fe_2O_3}{\text{excès de } K_2O} : \text{de 3/1 à 10/1 ; et}$$

$$\frac{CrO_3}{\text{excès de } K_2O} : \text{de 0,1/1 à 0,4/1 ;}$$

3

Les composés du chrome, utilisés seuls ou en mélange, sont en général l'anhydride chromique, les sels chromiques, par exemple chromate ou bichromate de sodium, de potassium ou d'ammonium, les composés du chrome $^+3$, par exemple nitrate, sulfate, oxyde ou hydroxyde.

Les composés utilisés pour introduire le potassium sont en général les carbonates, les oxydes et les hydroxydes. On peut encore utiliser d'autres sels, par exemple les sulfates, les phosphates ou les nitrates.

Des exemples de matériaux argileux utilisables consistent en kaolinite, halloïsite, bentonite, montmorillonite, attapulgite ou leurs mélanges. On utilisera de préférence la kaolinite, par exemple sous la forme de kaolin de Cornouailles.

Enfin, on pourra mettre en jeu une proportion mineure d'un agent favorisant l'extrusion. De tels matériaux sont connus de l'homme de l'art. On peut citer par exemple : le graphite, des gommes végétales (gomme arabique, gomme Damhar, gomme de Caroube) ou des alkylcelluloses (méthyl-éthyl- ou carboxyméthylcelluloses).

Les catalyseurs de l'invention préparés comme décrit ci-dessus, sont utilisés dans la déshydrogénation d'hydrocarbures, en particulier d'hydrocarbures aliphatiques saturés ou monoéthyléniques, et d'hydrocarbures alkylaromatiques (éthylbenzène, diéthylbenzènes), dans des conditions opératoires connues en soi et rappelées plus haut.

Dans la déshydrogénation de l'éthylbenzène en styrène, notamment, les catalyseurs de l'invention permettent d'obtenir une meilleure conversion de l'éthylbenzène et/ou une meilleure sélectivité en styrène.

Les exemples suivants illustrent l'invention. Ils ne doivent en aucune manière être considérés comme limitatifs. Les exemples 1, 2 et 4 à 9 sont donnés à titre de comparaison.

## Exemple 1 (Catalyseur A)

On malaxe dans un appareil WARRING-BLENDER, 300 g d'oxyde de fer ferrique « HA 160 », 14,5 g de bichromate de potassium, 100 g de carbonate de potassium anhydre et 27 g d'oxyde de lanthane $La_2O_3$.

Les quantités de réactifs utilisées correspondent, en oxydes, aux proportions suivantes (% poids) :

$Fe_2O_3$ = 73,23 %  
$CrO_3$ = 2,41 %  
$K_2O$ = 17,77 %  
$La_2O_3$ = 6,59 %

Les rapports pondéraux entre le fer, le chrome et le potassium, comptés en oxydes, sont les suivants :

$$\frac{Fe_2O_3}{K_2O} = 4,12 \; ; \quad \frac{CrO_3}{K_2O} = 0,135 \; ; \quad \frac{Fe_2O_3}{CrO_3} = 30,4$$

On ajoute ensuite 67 ml d'une solution aqueuse à 2 % en poids de Méthocel® (commercialisé par DOW CHEMICALS) et 70 ml d'eau, de façon à obtenir une pâte consistante, qui est malaxée durant 20 minutes puis transférée dans une extrudeuse à piston et extrudée en joncs de 4 mm de diamètre et de 2 à 6 mm de longueur.

L'analyse du catalyseur fini confirme les teneurs en oxydes de fer, de chrome, de potassium et de lanthane.

## Exemple 2 (Catalyseur B)

On malaxe dans un appareil WARRING-BLENDER, 300 g d'oxyde de fer ferrique « HA 160 », 14,5 g de bichromate de potassium, 100 g de carbonate de potassium anhydre, 17,9 g d'oxyde de lanthane $La_2O_3$ et 9,1 g d'oxyde de cobalt $Co_2O_3$.

Les quantités de réactifs mises en jeu correspondent en oxydes, aux pourcentages suivants (en % poids) :

$Fe_2O_3$ = 73,23 %  
$CrO_3$ = 2,41 %  
$K_2O$ = 17,77 %  
$La_2O_3$ = 4,37 %  
$Co_2O_3$ = 2,22 %

On ajoute 50 ml d'une solution aqueuse à 2 % en poids de Méthocel® et 100 ml d'eau de façon à obtenir une pâte qui est malaxée durant 15 minutes puis transférée dans une extrudeuse. L'extrusion, le séchage et l'activation du catalyseur sont effectués comme indiqué précédemment.

**0 112 240**

Exemple 3 (Catalyseur C)

145,5 g de nitrate de cobalt et 190 ml d'une solution aqueuse à 2,63 mole/l de nitrate de lanthane sont versés dans 2,5 l d'eau à 80 °C renfermant 212 g de $Na_2CO_3$. On obtient un précipité, qu'on lave par 12 litres d'eau distillée.

On laisse mûrir durant 6 heures à 80 °C, on filtre et l'on sèche 24 heures à 50 °C, 72 heures à 100 °C puis 24 heures à 120 °C. Enfin, on chauffe pendant 2 heures à 600 °C.

On mélange 27 g du produit ainsi obtenu avec 300 g d'oxyde de fer ferrique « HA 160 », 14,5 g de bichromate de potassium et 100 g de carbonate de potassium anhydre.

Les quantités de réactifs mises en jeu correspondent en oxydes, aux pourcentages suivants (% poids) :

$Fe_2O_3$ = 73,23 %
$CrO_3$ = 2,41 %
$K_2O$ = 17,77 %
$LaCoO_3$ = 6,59 %

On procède ensuite dans les mêmes conditions qu'indiqué précédemment.

Exemple 4 (Catalyseur D)

On malaxe dans un appareil WARRING-BLENDER, 300 g d'oxyde de fer ferrique « HA 160 », 14,5 g de bichromate de potassium, 100 g de carbonate de potassium anhydre, 28,5 g de Kaolin de Cornouailles et 27 g d'oxyde de lanthane $La_2O_3$. Les quantités de réactifs utilisées correspondent, en oxydes, aux pourcentages pondéraux suivants :

$Fe_2O_3$ = 68,47 %
$CrO_3$ = 2,25 %
$K_2O$ = 16,62 %
$La_2O_3$ = 6,16 %
Kaolin = 6,50 %

Les rapports pondéraux entre le fer, le chrome et le potassium comptés en oxydes, ont des valeurs voisines de celles indiquées dans l'exemple 1.

La quantité de potassium susceptible de se combiner avec le kaolin correspond à une proportion pondérale, en oxyde, de 2,76 % par rapport à l'ensemble des oxydes. La proportion d'oxyde de potassium « en excès » est donc de 13,86 %.

Les rapports entre l'oxyde de fer et l'oxyde de chrome d'une part, et l'excès de $K_2O$ d'autre part sont respectivement les suivants :

$$\frac{Fe_2O_3}{\text{excès de } K_2O} = 4,94 \; ; \quad \frac{CrO_3}{\text{excès de } K_2O} = 0,162$$

On ajoute ensuite 67 ml d'une solution aqueuse à 2 % en poids de Méthocel® (commercialisé par DOW CHEMICALS) et 70 ml d'eau, de façon à obtenir une pâte consistante.

On procède ensuite comme indiqué dans l'exemple 1.

L'analyse du catalyseur fini confirme sa teneur en oxydes de fer, de chrome, de potassium et de lanthane. L'analyse par diffraction des Rayons X, permet de mettre en évidence la présence de silicate double d'aluminium et de potassium (kaliophyllite) dont la teneur pondérale est de 9,3 %.

Exemples 5 et 6 (Catalyseurs E et F)

On répète la préparation décrite dans l'exemple 4, à la différence près que l'on utilise, au lieu de l'oxyde de lanthane $La_2O_3$, une même quantité pondérale d'un autre oxyde de terre rare :
exemple 5 : l'oxyde de cérium $CeO_2$ (Catalyseur E),
exemple 6 : l'oxyde de néodyme $NdO_3$ (Catalyseur F).

Exemple 7 (Catalyseur G)

On malaxe dans un appareil WARRING-BLENDER, 300 g d'oxyde de fer ferrique « HA 160 », 14,5 g de bichromate de potassium, 100 g de carbonate de potassium anhydre, 28,5 g de Kaolin de Cornouailles, 17,9 g d'oxyde de lanthane $La_2O_3$, 9,1 g d'oxyde de cobalt $Co_2O_3$. On a choisi les mêmes quantités atomiques de lanthane et de cobalt.

5

Les quantités de réactifs mises en jeu correspondent, en oxydes, aux pourcentages suivants (% poids) :

$Fe_2O_3$ = 68,47 %
$CrO_3$ = 2,25 %
$K_2O$ = 16,62 %
$La_2O_3$ = 4,08 %
$Co_2O_3$ = 2,08 %
Kaolin = 6,50 %

On ajoute 50 ml d'une solution aqueuse à 2 % en poids de Méthocel® et 100 ml d'eau de façon à obtenir une pâte qui est malaxée durant 15 minutes. L'extrusion, le séchage et la calcination du catalyseur sont effectués comme indiqué précédemment.

La teneur en silicate double d'aluminium et de potassium (kaliophyllite) dans les catalyseurs finis est voisine de 9,3 % en poids.

## Exemple 8 (Catalyseur H)

On répète la préparation décrite dans l'exemple 7 (Catalyseur G), à la différence près qu'au lieu d'oxyde de cobalt $Co_2O_3$, on utilise une même quantité pondérale d'anhydride vanadique, $V_2O_5$.

## Exemple 9 (Catalyseur I)

On répète la préparation décrite dans l'exemple 7 (Catalyseur G), excepté qu'au lieu d'oxyde de lanthane $La_2O_3$, on utilise une même quantité pondérale d'oxyde de cérium $CeO_2$, et qu'au lieu d'oxyde de cobalt $Co_2O_3$, on utilise une même quantité pondérale d'anhydride vanadique $V_2O_5$.

## Exemple 10 (Catalyseur J)

145,5 g de nitrate de cobalt et 190 ml d'une solution aqueuse à 2,63 mole/l de nitrate de lanthane sont versés dans 2,5 l d'eau à 80 °C renfermant 212 g de $Na_2CO_3$. Les quantités de réactifs mises en jeu correspondent à un rapport atomique du cobalt au lanthane de 1/1.

On obtient un précipité, qu'on lave par 12 litres d'eau distillée. On laisse mûrir durant 6 heures à 80 °C, on filtre et on sèche 24 heures à 50 °C, 72 heures à 100 °C puis 24 heures à 120 °C. Enfin, on chauffe pendant 2 heures à 600 °C.

La structure du produit obtenu n'est pas identifiée avec certitude. Il semble qu'il s'agit, au moins partiellement, d'un oxyde mixte du type perovskite $LaCoO_3$.

On mélange 27 g du produit ainsi préparé avec 300 g d'oxyde de fer ferrique « HA 160 », 14,5 g de bichromate de potassium, 100 g de carbonate de potassium anhydre et 28,5 g de kaolin de Cornouailles.

Les quantités de réactifs mises en jeu correspondent, en oxydes, aux pourcentages suivants : (% poids) :

$Fe_2O_3$ = 68,47 %
$CrO_3$ = 2,25 %
$K_2O$ = 16,62 %
$LaCoO_3$ = 6,16 %
Kaolin = 6,50 %

On procède ensuite dans les mêmes conditions qu'indiquées précédemment.

## Exemple 11 (Catalyseur K)

On fait réagir à l'état solide un mélange de 163 g d'oxyde de lanthane $La_2O_3$ et de 91 g d'anhydride vanadique $V_2O_5$, sous atmosphère d'hydrogène à 850 °C pendant 6 heures, puis on calcine 18 heures à 1 200 °C. Les oxydes mis en jeu en des proportions correspondant à un rapport atomique La/V d'environ 1/1.

On obtient un produit constitué au moins en partie d'oxyde mixte $LaVO_3$ (de structure perovskite). On procède ensuite comme décrit dans l'exemple 10.

## Exemple 12 (Catalyseur L)

On répète la préparation décrite dans l'exemple 11 (catalyseur K), à cela près qu'au lieu d'oxyde de lanthane $La_2O_3$, on utilise de l'oxyde de cérium $CeO_2$ en une quantité correspondant à un rapport atomique Ce/V d'environ 1/1.

On obtient un produit constitué au moins en partie d'oxyde mixte $CeVO_3$ (de structure perovskite). On procède ensuite comme décrit dans l'exemple 10.

Exemples 13 à 18 (Catalyseurs M à R)

On fait réagir en solution dans l'eau des sels de métaux de terres rares (TR) et des sels d'autres métaux (M) en des quantités correspondant à un rapport atomique tr/M d'environ 1/1.

On procède ensuite comme indiqué dans l'exemple 10.

Les produits obtenus peuvent être représentés par les formules figurant au tableau 1.

Tableau 1

| EXEMPLE N° | CATALYSEUR | OXYDE MIXTE |
|---|---|---|
| 13 | M | $LaCrO_3$ |
| 14 | N | $LaFeO_3$ |
| 15 | O | $LaCr_{0,6}Fe_{0,4}O_3$ |
| 16 | P | $CeCrO_3$ |
| 17 | Q | $CeFeO_3$ |
| 18 | R | $Nd\ Cr\ O_3$ |

Le reste de la préparation est identique à ce qui est décrit dans l'exemple 10.

L'analyse des catalyseurs obtenus comme décrit dans les exemples 1 à 18 indique que leur composition centésimale, en oxydes, est voisine de la composition des constituants mis en jeu pour leur préparation, en ce qui concerne le fer, le chrome, le potassium, le métal de terre rare (TR) et éventuellement le métal (M) associé. Par ailleurs, en ce qui concerne les exemples 4 à 18, l'analyse par diffraction des rayons X relève une teneur en silicate double d'aluminium et de potassium d'environ 9,3 % en poids.

Les catalyseurs A à R ont fait l'objet d'un test catalytique de longue durée.

Le test catalytique est effectué dans un « catatest » qui fonctionne sous pression atmosphérique et est alimenté en éthylbenzène industriel et en eau. Le volume de catalyseur testé est 100 ml (60 à 120 g cata). Les catalyseurs A à R sont sous la forme d'extrudés de 4 mm de diamètre et de 4-5 mm de longueur.

Le catalyseur est tout d'abord préchauffé jusqu'à environ 500 °C. On introduit alors la vapeur, puis, vers environ 550 °C, on introduit l'éthylbenzène. Les températures sont alors ajustées pour avoir une température de 614 ± 2 °C dans le lit catalytique.

Les débits horaires choisis sont les suivants :

$$\frac{Ethylbenzène}{catalyseur} = 0,4 \text{ volume } v^{-1}\ h^{-1} \qquad \frac{H_2O}{Ethylbenzène} = 2\ g \quad g^{-1}$$

On indique au tableau 2 la conversion de l'éthylbenzène ($C_{EB}$), la sélectivité en styrène ($S_{ST}$) et le rendement en styrène ($R_{ST}$) ; ces différents chiffres sont exprimés en % molaire et correspondent aux définitions suivantes :

$$C_{EB} = \frac{\text{moles d'éthylbenzène transformées}}{\text{moles d'éthylbenzène engagées}} \times 100$$

$$S_{ST} = \frac{\text{moles d'éthylbenzène transformées en styrène}}{\text{moles d'éthylbenzène transformées}} \times 100$$

$$R_{ST} = \frac{\text{moles de styrène produites}}{\text{moles d'éthylbenzène engagées}} \times 100$$

Ces grandeurs sont reliées par la relation :

$$R_{ST} = C_{EB} \times S_{ST} \times \frac{1}{100}$$

Les performances catalytiques sont rassemblées dans le tableau 2.

Tableau 2

| Catalyseur | A | B | C | D | E | F | G | H | I |
|---|---|---|---|---|---|---|---|---|---|
| Exemple | 1 | 2 | 3 | 4 | 5 | 6 | 7 | 8 | 9 |
| Convers.EB % mol. | 59,9 | 60,8 | 65,8 | 60,0 | 60,2 | 59,0 | 61,0 | 60,0 | 60,1 |
| Sélect. ST % mol. | 92,0 | 90,5 | 91,7 | 92,1 | 90,0 | 92,5 | 90,5 | 92,5 | 90,5 |
| Rendem. ST % mol. | 55,1 | 56,2 | 60,3 | 55,3 | 54,2 | 54,6 | 55,2 | 55,5 | 54,4 |
| Catalyseur | J | K | L | M | N | 0 | P | Q | R |
| Exemple | 10 | 11 | 12 | 13 | 14 | 15 | 16 | 17 | 18 |
| Convers.EB % mol. | 65,9 | 61,0 | 60,5 | 65,4 | 62,5 | 62,0 | 65,5 | 62,1 | 61,9 |
| Sélect. ST % mol. | 91,9 | 92,0 | 90,4 | 91,5 | 92,7 | 92,8 | 90,4 | 92,6 | 90,3 |
| Rendem. ST % mol. | 60,5 | 56,1 | 54,7 | 59,8 | 57,9 | 57,5 | 59,2 | 57,2 | 55,9 |

Dans ce tableau, la comparaison entre l'exemple 3 et l'exemple 2, la comparaison entre les exemples 13, 14 et 15 et l'exemple 4, la comparaison entre l'exemple 10 et l'exemple 7, la comparaison entre l'exemple 11 et l'exemple 8, la comparaison entre les exemples 16 et 17 et l'exemple 5, la comparaison entre l'exemple 18 et l'exemple 6, et la comparaison entre l'exemple 12 et l'exemple 9 montrent que le mode de préparation de l'invention, comprenant une étape préalable de formation d'une association d'oxyde d'un métal de terre rare avec un oxyde d'un autre métal (M) (avec calcination intermédiaire), confère aux catalyseurs des propriétés améliorées par rapport aux catalyseurs obtenus lorsque toutes les espèces métalliques sont introduites en une seule étape.

Exemples 19 à 32

De la même manière que pour les exemples 10 à 18, on a préparé diverses autres combinaisons d'oxydes entre d'une part au moins un métal de terre rare et d'autre part au moins un métal M choisi parmi le fer, le chrome, le cobalt, l'aluminium et le vanadium. Les combinaisons d'oxydes peuvent être assimilées au moins en partie à des oxydes mixtes de type perovskite répondant aux formules figurant dans le tableau 3 ci-après.

(Voir Tableau 3 p. 9)

8

Tableau 3

| EXEMPLE N° | CATALYSEUR | OXYDE MIXTE |
|---|---|---|
| 19 | S | $La\ AlO_3$ |
| 20 | T | $La_{0,5}\ Pr_{0,5}\ Co\ O_3$ |
| 21 | U | $La_{0,7}\ Nd_{0,3}\ Fe_{0,5}\ Co_{0,5}\ O_3$ |
| 22 | V | $Pr\ Al\ O_3$ |
| 23 | W | $Pr\ Co_{0,1}\ Fe_{0,9}\ O_3$ |
| 24    * | X | $Pr_{0,8}\ Sc_{0,2}\ V\ O_3$ |
| 25 | Y | $Nd\ Al_{0,3}\ Co_{0,7}\ O_3$ |
| 26 | Z | $Sm\ Fe\ O_3$ |
| 27 | AA | $Sm\ Cr_{0,8}\ Co_{0,2}\ O_3$ |
| 28 | AB | $Eu\ Cr\ O_3$ |
| 29 | AC | $Gd\ Co\ O_3$ |
| 30    * | AD | $Gd\ Fe_{0,5}\ V_{0,5}\ O_3$ |
| 31 | AE | $Dy\ Al\ O_3$ |
| 32 | AF | $Yb\ Cr_{0,4}\ Fe_{0,4}\ Co_{0,2}\ O_3$ |

\* Dans les exemples 24 et 30, on a opéré de la même manière que dans l'exemple 11.

Le reste de la préparation est identique à ce qui a été décrit dans l'exemple 10.

Les catalyseurs ainsi formés ont été testés dans les conditions décrites précédemment. Leurs performances sont rassemblées dans le tableau 4. Les rendements en styrène observés sont particulièrement élevés.

Tableau 4

| Catalyseur | S | T | U | V | W | X | Y |
|---|---|---|---|---|---|---|---|
| Exemple | 19 | 20 | 21 | 22 | 23 | 24 | 25 |
| Convers.EB % mol. | 64,0 | 63,7 | 62,0 | 65,4 | 61,8 | 62,2 | 62,0 |
| Sélect. ST % mol. | 90,6 | 92,1 | 90,5 | 91,9 | 93,4 | 92,1 | 92,2 |
| Rendem. ST % mol. | 58,0 | 58,7 | 56,1 | 60,1 | 57,7 | 57,3 | 57,2 |
| Catalyseur | Z | AA | AB | AC | AD | AE | AF |
| Exemple | 26 | 27 | 28 | 29 | 30 | 31 | 32 |
| Convers.EB % mol. | 64,0 | 65,8 | 64,1 | 62,2 | 62,3 | 67,2 | 65,5 |
| Sélect. ST % mol. | 91,9 | 91,2 | 90,8 | 92,2 | 92,1 | 90,8 | 93,2 |
| Rendem. ST % mol. | 58,8 | 60,0 | 58,2 | 57,3 | 57,4 | 61,0 | 61,0 |

## Revendications

1. Procédé de préparation d'un catalyseur renfermant, sous forme d'oxydes, du fer, du chrome et du potassium, dans les rapports pondéraux :

$$\frac{Fe_2O_3}{K_2O} \quad de \quad 1/1 \ à \ 10/1$$

$$\frac{CrO_3}{K_2O} \quad de \ 0,05/1 \ à \ 0,4/1$$

$$\frac{Fe_2O_3}{CrO_3} \quad de \quad 15/1 \ à \ 40/1 \ ,$$

et au moins un métal de terre rare, en une proportion comptée en oxyde, de 1 à 15 % en poids par rapport au poids dudit catalyseur, ledit procédé étant caractérisé en ce qu'il comprend :

une première phase au cours de laquelle on prépare un produit par réaction d'au moins un composé de métal de terre rare (TR) avec au moins un composé d'au moins un métal (M) choisi parmi le fer, le chrome, le cobalt, l'aluminium et le vanadium, cette réaction étant suivie d'un chauffage à température élevée, le produit ainsi préparé à l'issue de cette première phase étant constitué au moins en partie d'un oxyde de type perovskite, et

une seconde phase au cours de laquelle on constitue un mélange comprenant de l'eau, au moins un composé du fer, au moins un composé du chrome, au moins un composé du potassium et le produit préparé à l'issue de la première phase, les divers composés de métaux étant mis en jeu dans des proportions correspondant aux proportions pondérales d'oxydes indiquées, on malaxe ledit mélange pour former une pâte qui est ensuite séchée et on active thermiquement le catalyseur par chauffage à température élevée.

2. Procédé selon la revendication 1, caractérisé en ce que, dans le catalyseur, les rapports pondéraux entre le fer, le chrome et le potassium, comptés en oxydes, sont :

$$\frac{Fe_2O_3}{K_2O} \text{ de 2/1 à 7/1 ;}$$

$$\frac{CrO_3}{K_2O} \text{ de 0,1/1 à 0,3/1 ; et}$$

$$\frac{Fe_2O_3}{CrO_3} \text{ de 25/1 à 35/1,}$$

et la proportion de métal de terre rare, comptée en oxyde, est de 3 à 10 % en poids.

3. Procédé selon la revendication 1 ou 2, caractérisé en ce que le métal de terre rare est le lanthane, le praséodyme ou le néodyme.

4. Procédé selon l'une des revendications 1 à 3, caractérisé en ce que les composés de métal de terre rare (TR) et de métal M sont mis en jeu en des proportions correspondant à un rapport atomique TR/M d'environ 0,5/1 à 2/1.

5. Procédé selon la revendication 4, caractérisé en ce que ledit rapport atomique TR/M est d'environ 1/1.

6. Procédé selon l'une des revendications 1 à 5, caractérisé en ce que, dans la première phase, on effectue une réaction en solution entre au moins un composé soluble d'un métal de terre rare et au moins un composé soluble de fer, de chrome, de cobalt ou d'aluminium, on sépare le précipité obtenu, on le sèche et on le chauffe à une température de 400 à 950 °C.

7. Procédé selon l'une des revendications 1 à 5, caractérisé en ce que, dans la première phase, la réaction est effectuée entre oxydes ou composés décomposables en oxydes, d'au moins un métal de terre rare d'une part et d'au moins un métal M d'autre part par chauffage à une température de 900 à 1 250 °C.

8. Procédé selon l'une des revendications 1 à 5, caractérisé en ce que, dans la première phase, on effectue une réaction entre oxydes ou composés décomposables en oxydes, d'au moins un métal de terre rare d'une part et de vanadium d'autre part, sous atmosphère réductrice et à une température de 600 à 900 °C, puis on calcine à une température de 900 à 1 250 °C.

9. Procédé selon l'une des revendications 1 à 8, caractérisé en ce que au cours de la seconde phase, on met en jeu un matériau argileux en une proportion de 3,5 à 30 % en poids par rapport à l'ensemble des constituants dudit mélange, comptés en oxydes, le composé du potassium étant mis en jeu en excès par rapport à la quantité susceptible de se combiner avec ledit matériau argileux pour former un silicate double d'aluminium et de potassium, l'excès dudit composé de potassium étant avec le fer et le chrome, comptés en oxydes, dans les rapports pondéraux :

$$\frac{Fe_2O_3}{\text{Excès de } K_2O} \text{ de 3/1 à 10/1 ; et } \frac{CrO_3}{\text{Excès de } K_2O} : \text{ de 0,1/1 à 0,4/1}$$

10. Procédé selon la revendication 9, caractérisé en ce que ledit matériau argileux est constitué au moins en partie de kaolinite.

11. Procédé selon l'une des revendications 1 à 10, caractérisé en ce que l'activation thermique du catalyseur à la fin de la seconde phase est effectué à une température de 850 à 1 100 °C.

12. Catalyseur préparé par un procédé selon l'une des revendications 1 à 11.

13. Utilisation d'un catalyseur préparé selon le procédé de l'une des revendications 1 à 11, dans une réaction de déshydrogénation d'un hydrocarbure alkylaromatique.

14. Utilisation selon la revendication 13, dans laquelle ledit hydrocarbure alkylaromatique à déshydrogéner est l'éthylbenzène.

**Claims**

1. A process for manufacturing a catalyst containing iron, chromium and potassium, as oxides, in the weight ratios

$$\frac{Fe_2O_3}{K_2O} \text{ from 1/1 to 10/1}$$

$$\frac{CrO_3}{K_2O} \text{ from 0.05/1 to 0.4/1}$$

$$\frac{Fe_2O_3}{CrO_3} \text{ from 15/1 to 40/1}$$

and at least one rare earth metal, in a proportion, calculated as oxide, from 1 to 15 % by weight with respect to the weight of said catalyst, said process being characterized in that it comprises :

a first step in which a product is prepared by reacting at least one rare earth metal (TR) compound with at least one compound of at least one metal (M) selected from iron, chromium, cobalt, aluminum and vanadium, said reaction being followed with heating at high temperature, the product so prepared at the end of this first step being comprised at least partly of an oxide of the perovskite type ; and

a second step in which a mixture is formed which comprises water, at least one iron compound, at least one chromium compound, at least one potassium compound and the product obtained at the end of the first step, the various metal compounds being used in proportions corresponding to the oxide weight proportions indicated, said mixture is malaxated to form a paste which is then dried and the catalyst is thermally activated by heating at high temperature.

2. A process according to claim 1, characterized in that, in the catalyst, the weight ratios between iron, chromium and potassium, calculated as oxides, are :

$$\frac{Fe_2O_3}{K_2O} \text{ from 2/1 to 7/1}$$

$$\frac{CrO_3}{K_2O} \text{ from 0.1/1 to 0.3/1 and}$$

$$\frac{Fe_2O_3}{CrO_3} \text{ from 25/1 to 35/1}$$

and the proportion of rare earth metal, calculated as oxide, is from 3 to 10 % by weight.

3. A process according to claim 1 or 2, characterized in that the rare earth metal is lanthanum, praseodymium or neodymium.

4. A process according to one of claims 1 to 3, characterized in that the rare earth metal (TR) and metal M compounds are used in proportions corresponding to an atomic ratio RE/M of from about 0.5/1 to 2/1.

5. A process according to claim 4, characterized in that said atomic ratio RE/M is about 1/1.

6. A process according to one of claims 1 to 5, characterized in that, in the first step, the reaction is carried out in solution between at least a soluble compound of a rare earth metal and at least a soluble compound of iron, chromium, cobalt or aluminum, the resulting precipitate is separated, dried, and heated at a temperature of from 400° to 950 °C.

7. A process according to one of claims 1 to 5, characterized in that in the first step the reaction is carried out between oxides or species that thermally decompose to oxides, of said at least one rare earth metal on the one hand and at least one metal M on the other hand, under heating a temperature of from 900° to 1 250 °C.

8. A process according to one of claims 1 to 5, characterized in that, in the first step, the reaction is carried out by mixing oxides, or species that thermally decompose to oxides, of at least one rare earth metal, on the one hand, and vanadium, on the other hand, under a reducing atmosphere and at a temperature of from 600° to 900 °C, and there is calcined at a temperature of from 900° to 1 250 °C.

9. A process according to one of claims 1 to 8, characterized in that, in the second step, a clayish material is used in a proportion from 3.5 to 30 % by weight with respect to the totality of the mixture components, calculated as oxides, the potassium compound being used in excess with respect to the amount liable to combine with said clayish material to form a double aluminum and potassium silicate, the excess of said potassium compound being, in proportion to iron and chromium, calculated as oxides, in the weight ratios :

$$\frac{Fe_2O_3}{K_2O \text{ excess}} : \text{from 3/1 to 10/1 ; and}$$

$$\frac{CrO_3}{K_2O \text{ excess}} : \text{from 0.1/1 to 0.4/1}$$

10. A process according to claim 9, characterized in that said clayish material is comprised at least partly of kaolinite.

11. A process according to one of claims 1 to 10, characterized in that the thermal activation of the catalyst at the end of the second step is effected at a temperature from 850° to 1 100 °C.

12. A catalyst prepared by a process according to one of claims 1 to 11.

13. The use of a catalyst prepared according to the process of one of claims 1 to 11, in a dehydrogenation reaction of an alkylaromatic hydrocarbon.

14. A use according to claim 13, in which said alkylaromatic hydrocarbon to be dehydrogenated is ethylbenzene.

**Patentansprüche**

1. Verfahren zur Herstellung eines Katalysators, der, in Form der Oxyde, Eisen, Chrom und Kalium in folgenden Gewichtsverhältnissen enthält :

$$\frac{Fe_2O_3}{K_2O} \text{ von 1/1 bis 10/1}$$

$$\frac{CrO_3}{K_2O} \text{ von 0,05 bis 0,4/1}$$

$$\frac{Fe_2O_3}{CrO_3} \text{ von 15/1 bis 40/1}$$

und mindestens eine Seltenes Erdmetall in einem Mengenanteil, berechnet als Oxyd, von 1 bis 15 Gew.-% bezogen auf das Gewicht des Katalysators, gekennzeichnet durch :

eine erste Phase im Verlaufe welcher man ein Produkt durch Umsetzung von zumindest einer Verbindung des seltenen Erdmetalls (TR) mit zumindest einer Verbindung zumindest eines Metalls (M) der Gruppe Eisen, Chrom, Kobalt, Aluminium und Vanadium herstellt, wobei auf diese Reaktion ein Erhitzen auf erhöhte Temperatur folgt und das so am Ende dieser ersten Phase hergestellte Produkt zumindest zum Teil aus einem Oxyd vom Typ Perovskit besteht, und

eine zweite Phase im Verlaufe welcher man ein Gemisch bildet, das Wasser, zumindest eine Verbindung von Eisen, zumindest eine Verbindung von Chrom, zumindest eine Verbindung von Kalium und das am Ende der ersten Stufe hergestellte Produkt enthält, wobei die verschiedenen Metallverbindungen in Verhältnissen eingesetzt werden, welche den angegebenen Gewichtsanteilen der Oxyde entsprechen, man diese Mischung unter Bildung einer Paste mischt bzw. knetet, die dann anschließend getrocknet wird und den Katalysator durch Erhitzen auf erhöhte Temperatur thermisch aktiviert.

2. Verfahren nach Anspruch 1, dadurch gekennzeichnet, daß im Katalysator die Gewichtsverhältnisse zwischen Eisen, Chrom und Kalium, berechnet als Oxyde, wie folgt sind :

$$\frac{Fe_2O_3}{K_2O} \text{ von 2/1 bis 7/1 ;}$$

$$\frac{CrO_3}{K_2O} \text{ von 0,1/1 bis 0,3/1 ; und}$$

$$\frac{Fe_2O_3}{CrO_3} \text{ von 25/1 bis 35/1}$$

und die Menge des Seltenen Erdmetalles, berechnet als Oxyd, 3 bis 10 Gew.-% beträgt.

3. Verfahren nach Anspruch 1 oder 2, dadurch gekennzeichnet, daß das Seltene Erdmetall Lanthan, Praseodym oder Neodym ist.

4. Verfahren nach einem der Ansprüche 1 bis 3, dadurch gekennzeichnet, daß die Verbindungen des Seltenen Erdmetalls (TR) und des Metalles M in Mengenanteilen eingesetzt werden, welche einem Atomverhältnis TR/M von etwa 0,5/1 bis 2/1 entsprechen.

5. Verfahren nach Anspruch 4, dadurch gekennzeichnet, daß dieses Atomverhältnis TR/M etwa 1/1 beträgt.

6. Verfahren nach einem der Ansprüche 1 bis 5, dadurch gekennzeichnet, daß in der ersten Phase man eine Reaktion in Lösung zwischen zumindest einer löslichen Verbindung eines seltenen Erdmetalls und zumindest einer löslichen Verbindung von Eisen, Chrom, Kobalt oder Aluminium bewirkt, den erhaltenen Niederschlag abtrennt, ihn trocknet und auf eine Temperatur von 400 bis 950 °C erhitzt.

7. Verfahren nach einem der Ansprüche 1 bis 5, dadurch gekennzeichnet, daß in der ersten Stufe die Reaktion zwischen den Oxiden oder zu Oxiden zersetzbaren Verbindungen von zumindest einem Seltenen Erdmetall einerseits und zumindest einem Metall M andererseits durch Erhitzen auf eine Temperatur von 900 bis 1 250 °C bewirkt wird.

8. Verfahren nach einem der Ansprüche 1 bis 5, dadurch gekennzeichnet, daß in der ersten Stufe eine Reaktion zwischen Oxiden oder zu Oxiden zersetzbaren Verbindungen zumindest eines Seltenen Erdmetalls einerseits und Vanadium andererseits unter reduzierender Atmosphäre und bei Temperatur von 600 bis 900 °C durchgeführt wird und dann bei einer Temperatur von 900 bis 1 250 °C calciniert wird.

9. Verfahren nach einem der Ansprüche 1 bis 8, dadurch gekennzeichnet, daß man im Verlaufe der zweiten Phase ein Tonmaterial in einer Menge von 3,5 bis 30 Gew.-%, bezogen auf die gesamten Bestandteile dieser Mischung, berechnet als Oxide einsetzt und die Kaliumverbindung im Überschuß, bezogen auf die Menge, die zur Kombination mit diesem Tonmaterial zur Bildung eines Doppelsilikates von Aluminium und Kalium befähigt ist, eingesetzt wird, wobei der Überschuß dieser Kaliumverbindung mit Eisen und Chrom, berechnet als Oxide, in den folgenden Gewichtsverhältnissen vorliegt :

$$\frac{Fe_2O_3}{\text{Überschuß an } K_2O} : \text{von 3/1 bis 10/1 ; und}$$

$$\frac{CrO_3}{\text{Überschuß an } K_2O} : \text{von 0,1/1 bis 0,4/1}$$

10. Verfahren nach Anspruch 9, dadurch gekennzeichnet, daß dieses Tonmaterial zumindest zum Teil aus Kaolinit besteht.

11. Verfahren nach einem der Ansprüche 1 bis 10, dadurch gekennzeichnet, daß die thermische Aktivierung des Katalysators am Ende der zweiten Phase bei einer Temperatur von 850 bis 1 100 °C durchgeführt wird.

12. Katalysator, hergestellt nach einem Verfahren gemäß einem der Ansprüche 1 bis 11.

13. Verwendung eines Katalysators, hergestellt gemäß einem Verfahren nach einem der Ansprüche 1 bis 11, für die Reaktion der Dehydrierung eines alkylaromatischen Kohlenwasserstoffs.

14. Verwendung nach Anspruch 13, dadurch gekennzeichnet, daß dieser zu dehydrierende alkylaromatische Kohlenwasserstoff Äthylbenzol ist.